# EUROPÄISCHE PATENTSCHRIFT

(11) **EP 1 000 016 B1**
(45) Veröffentlichungstag und Bekanntmachung des Hinweises auf die Patenterteilung: **28.01.2004**
(21) Anmeldenummer: 98938702.2
(22) Anmeldetag: 23.07.1998
(51) Int. Cl.: C07C 235/08, C07C 237/08, C07C 291/10, C07D 295/18

(54) **ISOCYANOALKYLKOHLENSÄUREDERIVATE, DEREN UMSETZUNG IN ISOCYANID-MULTIKOMPONENTENREAKTIONEN ZU SEK.AMIDOALKYLKOHLENSÄUREDERIVATEN UND DIESE SEK.AMIDOALKYLKOHLENSÄUREDERIVATE**
ISOCYANOALKYL CARBONIC ACID DERIVATIVES, THEIR CONVERSION INTO SECONDARY AMIDOALKYL CARBONIC ACID DERIVATIVES BY ISOCYANIDE-MULTICOMPONENT REACTIONS, AS WELL AS THESE SECONDARY AMIDOALKYL CARBONIC ACID DERIVATIVES.
DERIVES D'ACIDE ISOCYANOALKYLECARBONIQUE, CONVERSION DESDITS DERIVES EN DERIVES D'ACIDE AMIDOALKYLECARBONIQUE SECONDAIRES A L'AIDE DE REACTIONS D'ISOCYANURE A CONSTITUANTS MULTIPLES ET LESDITS DERIVES SECONDAIRES

(30) Priorität: 25.07.1997 DE 19732176; 25.07.1997 DE 19731893
(43) Veröffentlichungstag der Anmeldung: 17.05.2000
(73) Patentinhaber: Ugichem GmbH, 6020 Innsbruck (AT)
(72) Erfinder: UGI, Ivar, D-85747 Garching (DE); BOCK, Holger, D-80807 München (DE); LINDHORST, Thomas, D-81377 München (DE)
(74) Vertreter: Forstmeyer, Dietmar, Dr. rer. nat., Dipl.-Chem.
(86) Internationale Anmeldenummer: PCT/EP1998/004622
(87) Internationale Veröffentlichungsnummer: WO 1999/005093

(56) Entgegenhaltungen:
- ILSE HAGEDORN ET AL.: "Einstufige Synthese von alpha-Hydroxysäure-amiden durch Abwandlung der Passerini-Reaktion" CHEMISCHE BERICHTE, Bd. 98, Nr. 3, 1965, Seiten 936-940, XP002084700 WEINHEIM DE in der Anmeldung erwähnt
- KONSTANTINA KEHAGIA ET AL.: "The Formation of beta-Lactam Derivatives and a C3-Symmetrical Heterocycle from 5,6-Dihydro-2H-1,3-oxazines" TETRAHEDRON, Bd. 51, Nr. 1, 2. Januar 1995, Seiten 139-144, XP002084701 OXFORD GB in der Anmeldung erwähnt
- IVAR UGI ET AL.: "Ugi Reactions with Trifunctional alpha-Aminoacids, Aldehydes, Isocyanides and Alcohols" TETRAHEDRON, Bd. 52, Nr. 35, 26. August 1996, Seiten 11657-11664, XP002084702 OXFORD GB in der Anmeldung erwähnt

## Beschreibung

Die vorliegende Erfindung betrifft Isocyanoalkylkohlensäurederivate, deren Umsetzung in Isocyanid-Multikomponentenreaktionen zu sek.Amidoalkylkohlensäurederivaten und diese sek.Amidoalkylkohlensäurederivate.

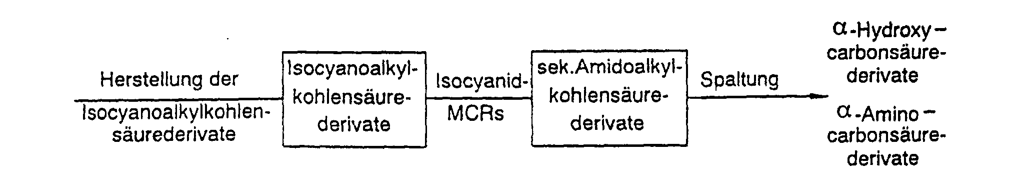

Viele wirtschaftlich interessante, organische Substanzen (z.B. pharmakologische Wirkstoffe und Naturstoffe) beinhalten Strukturmerkmale, die von α-Hydroxycarbonsäure- oder α-Aminocarbonsäurederivaten abgeleitet sind. Die Synthese dieser Substanzen ist oft ein aufwendiger Prozeß. Mit Hilfe von Isocyanid-Multikomponentenreaktionen (Isocyanid-MCRs) wie der Passerini-Reaktion (P-3CR) und den verschiedenen Varianten der Ugi-Reaktion können diese komplizierten Strukturmerkmale einfach hergestellt werden. [I. Ugi et al in: *Comprehensive Organic Synthesis: Selectivity for Synthetic Efficiency, Band II,* B. M. Trost, C.H. Heathcock, Pergamon Press, Oxford, **1991]**

Bei Multikomponentenreaktionen können drei oder mehr Edukte (Komponenten) simultan oder nahezu simultan in einer Eintopfreaktion miteinander umgesetzt werden, ohne daß eine Aufarbeitung von Zwischenprodukten erforderlich wäre. Zu der Gruppe der Isocyanid-MCRs (eine Komponente ist ein Isocyanid) gehören z.B. die Passerini-3-Komponentenreaktion (P-3CR), die Ugi-4-Komponentenreaktion (U-4CR) [I. Ugi, *Angew. Chem.* **1962,** *74,* 9;] und die Ugi-5-Zentren-4-Komponentenreaktion (U-5C-4CR) [I. Ugi et al, *Angew. Chem*. **1996,** *108,* 185] und die Ugi-4-Komponentenreaktion mit β-Aminosäuren (U-4CR/β-AS) [I. Ugi et al, *Tetrahedron* **1995,** *51,* 139].

Die Produkte dieser vier Reaktionstypen weisen sekundäre Amidfunktionen auf, die nur schwer in andere Derivate wie Carbonsäuren und Carbonsäureester überführt werden können, ohne daß dabei der Rest des Moleküls oder vorhandene Stereozentren zerstört werden. Das Kohlenstoffatom der gebildeten sekundären Amidfunktion stammt aus der eingesetzten Isocyanidkomponente (R^{Iso}-NC).

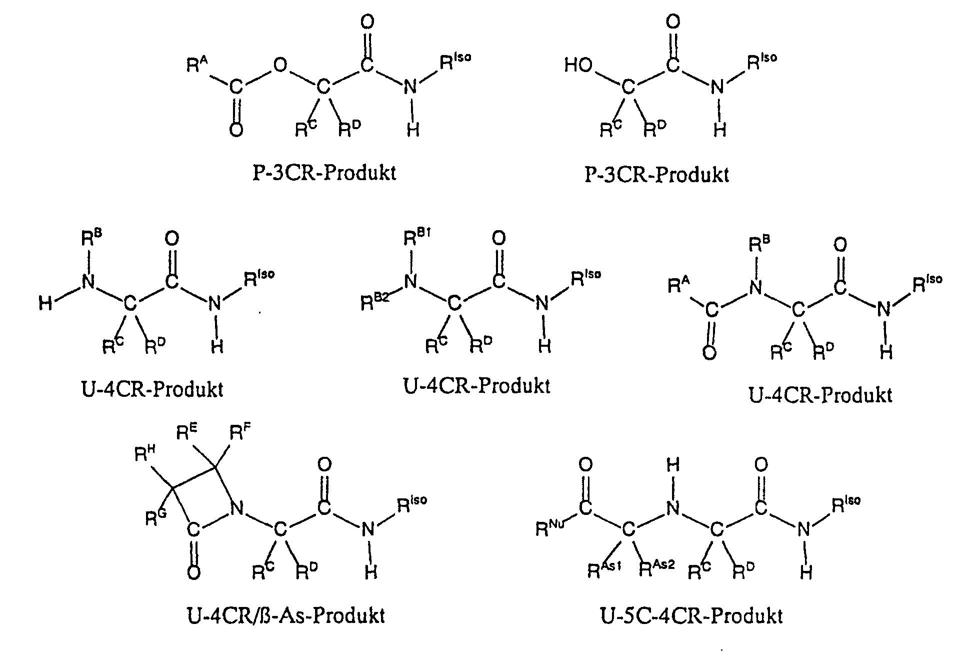

Es ist daher die Aufgabe der vorliegenden Erfindung, neue Isocyanid-MCR-Produkte (sek.Amidoalkylkohlensäurederivate) bereitzustellen, deren sekundäre Amidfunktion unter sehr milden Reaktionsbedingungen in wirtschaftlich interessante Produkte (pharmakologische Teilstrukturen) wie α-Hydroxycarbonsäure- oder α-Aminocarbonsäurederivate überführt werden kann.

Es ist eine weitere Aufgabe der vorliegenden Erfindung, Verfahren und Zwischenprodukte zur Herstellung der Isocyanid-MCR-Produkte bereitzustellen.

Diese Aufgabe wird durch Verbindungen (Isocyanoalkylkohlensäurederivate) der allgemeinen Formel I gelöst

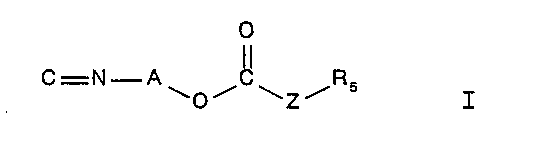

Die Isocyanoalkylkohlensäurederivate I sind zumindest bifunktionelle Moleküle, bei denen eine Isocyanidfunktion und eine Kohlensäurederivatfunktion über eine Brücke A, die eine Kettenlänge von 2-3 Kohlenstoffatomen aufweist, miteinander verbunden sind. Von besonderer Bedeutung ist, daß die Verbindung I nur eine Isocyanidfunktion enthält: die Spaltung von sek.Amidoalkylkohlensäurederivaten, die durch Isocyanid-MCRs aus Diisocyanoalkylkohlensäurederivaten erhalten würden, würde zu uneinheitlichen Produkten führen.

In Formel I ist
A eine Gruppe der Formel 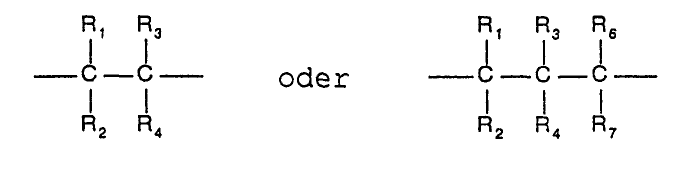 Z kann ein O-, oder ein S-Atom, vorzugsweise ein O-Atom sein.

Die Reste R₁ bis R₄, bzw. R₆ und R₇ umfassen jeweils maximal 20 C-Atome und sind unabhängig voneinander H-Atome, substituierte oder unsubstituierte Alkyl-, Alkenyl-, Alkinyl-, Alkaryl-, Aryl-, alicyclische Reste, heteroalicyclische oder Heteroarylreste mit bis zu 4 Heteroatomen (Heteroatome sind O, N, S), vorzugsweise umfassen diese Reste jeweils maximal 15, besonders bevorzugt maximal 10 C-Atome und sind unabhängig voneinander H-Atome, substituierte oder unsubstituierte Alkyl-, Alkenyl-, Alkinyl-, alicyclische oder Arylreste, stärker bevorzugt H-Atome, substituierte oder unsubstituierte Methyl-, Ethyl-, Propyl-, Butyl-, Pentyl- Hexyl-, Benzyl- und Cyclohexylreste, und am stärksten bevorzugt H-Atome, Methyl-, Ethyl-, Propyl-, Butyl-, Pentyl- Hexyl-, Benzyl- und Cyclohexylreste.

Zwei der Reste R₁ bis R₄ und gegebenenfalls R₆ und R₇, die durch bis zu zwei Kohlenstoffatome voneinander getrennt sind (wie z. B. die Reste R₁ und R₂, R₃ und R₄, R₁ und R₃ oder R₆ und R₇, aber nicht z.B. R₁ und R₆, da diese durch drei Köhlenstoffatome voneinander getrennt sind), können Bestandteile eines gemeinsamen Ringsystems sein, wobei das Ringsystem einen substituierten oder unsubstituierten alicyclischen Monocyclus (5-8 Ringatome), einen heteroalicyclischen Monocyclus (5-8 Ringatome mit 0, 1 oder 2 Heteroatomen [O, N, S]), einen alicyclischen Bicyclus (7-14 Ringatome), oder einen heteroalicyclischen Bicyclus (7-14 Ringatome mit bis zu 4 Heteroatomen [O, N, S]), vorzugsweise einen substituierten oder unsubstituierten alicyclischen Monocyclus (5-8 Ringatome), oder einen alicyclischen Bicyclus (7-14 Ringatome), am stärksten bevorzugt einen Cyclopentyl-, Cyclohexyl-, Norbornyl-, oder einen Bornylring umfaßt;

Der Rest R₅ umfaßt maximal 20, vorzugsweise 15, besonders bevorzugt 10 C-Atome und ist ein substituierter oder unsubstituierter Alkyl-, Alkenyl-, Alkinyl-, Allyl-, Alkaryl-, Aryl-, alicyclischer Rest, heteroalicyclischer oder Heteroarylrest mit bis zu 4 Heteroatomen (Heteroatome sind O, N, S), vorzugsweise ein Allyl-, Benzyl-, 2-Bromethyl-, n-Butyl-, 2-Chlorethyl-, 1-Chlorethyl-, Chlormethyl-, Ethyl-, 1-(9-Fluorenyl)-ethyl-, 9-Fluorenylmethyl-, isoButyl-, Menthyl-, 4-Methoxycarbonylphenyl, 4-Methoxyphenyl-, Methyl-, 4-Nitrobenzyl-, 2-Nitrophenyl-, 4-Nitrophenyl-, 4,5-Dimethoxy-2-nitrobenzyl-, Phenyl-, 2,2,2-Trichlor-tert.butyl-, 2,2,2-Trichlorethyl-, Vinyl-, 4-Methylphenyl-, 4-Fluorphenyl-, 4-Chlorphenyl-, 4-Bromphenyl-, α-Chloro-(trifluormethyl)benzyl-, 3-tert.Butylphenyl-, 2,4,6-Trichlorphenyl-, Pentafluorphenyl-, 2-(p-Toluolsulfonyl)ethyl-, Diphenylmethyl-, 2-(Trimethylsilyl)ethyl-, Methoxymethyl-, Methylthiomethyl-, (2-Trimethylsilyl)ethoxymethyl-, Benzyloxymethyl- oder ein (2-Methoxy)ethyloxymethylrest.

Gegebenenfalls können die Verbindungen der Formel I über einen oder mehrere der Reste R₁ bis R₄, bzw. R₆ und R₇, sowie R₅ oder Z an eine feste Phase (Harz) gebunden sein. Als Harze eignen sich z.B. alle konventionellen Harze, die in der organischen Festphasensynthese angewendet werden, wie z.B. Polystyroldivinylbenzol-, Polyethylenglycol- oder Polyethylen-glycolpolystyrol-Harze.

Die Reste R₁ bis R₄, bzw. R₆ und R₇, sowie R₅ und auch von ihnen gebildete Ringsysteme können mit einer, zwei, drei oder mehreren, vorzugsweise mit maximal einer der folgenden Gruppen substituiert sein:

Halogen-, tert.Amino-, Nitro-, Cyano-, Ether-, Silyloxyalkyl-, Thioether-, Carbonsäureester-, tert.Carbonsäureamid-, Carbonsäureimid-, Silyl-, Sulfonsäureester-, Sulfensäureester-, Sulfinsäureester-, tert.Sulfonamido-Funktion.

Die Substituenten der Reste R₁ bis R₄, bzw. R₆ und R₇ können z.B. die Acidität des zur Isocyanidfunktion α-ständigen H-Atoms erhöhen und ermöglichen somit den Einsatz von schwachen Basen bei der Synthese der Verbindung I. Die Wahl der Reste R₁ bis R₇ ist nicht wesentlich für die Eignung der Verbindung I für den Einsatz in den Isocyanid-MCRs und der anschließenden Spaltung (s.u.). Sie können aber z.B. dazu dienen, das Löslichkeitsverhalten der Isocyanoalkylkohlensäurederivate bzw. der bei der Spaltung entstehenden cyclischen Urethane zu steuern.

Insbesondere kann A eine Gruppe der Formel -C(CH₃)₂-CH₂- sein.

Bevorzugte Verbindungen der Formel I sind:
(2-Isocyano-2-methyl)-propyl-1-kohlensäureallylester, (2-Isocyano-2-methyl)-propyl-1-kohlensäurebenzylester, (2-Isocyano-2-methyl)-propyl-1-kohlensäure-(4-nitrobenzyl)ester, (2-Isocyano-2-methyl)-propyl-1-kohlensäureethylester, (2-Isocyano-2-methyl)-propyl-1-kohlensäurephenylester, (2-Isocyano-2-methyl)-propyl-1-kohlensäure-(4-nitrophenyl)ester, (2-Isocyano-2-methyl)-propyl-1-kohlensäurevinylester, (2-Isocyano-2-methyl)-propyl-1-kohlensäure-(2,2,2-trichlorethyl)-ester, (2-Isocyano-2-methyl)-propyl-1-kohlensäure-(2-trimethylsilyl-ethyl)ester, (2-Isocyano-2-methyl)-propyl-1-kohlensäuremethylester, (1-Isocyano-2-methyl)-propyl-2-kohlensäureallylester, (1-Isocyano-2-methyl)-propyl-2-kohlensäurebenzylester, (1-Isocyano-2-methyl)-propyl-2-kohlensäure-(4-nitrobenzyl)ester, (1-Isocyano-2-methyl)-propyl-2-kohlensäureethylester, (1-Isocyano-2-methyl)-propyl-2-kohlensäurephenylester, (1-Isocyano-2-methyl)-propyl-2-kohlensäure-(4-nitrophenyl)ester, (1-Isocyano-2-methyl)-propyl-2-kohlensäurevinylester, (1-Isocyano-2-methyl)-propyl-2-kohlensäure-(2,2,2-trichlorethyl)-ester, (1-Isocyano-2-methyl)-propyl-2-kohlensäure-(2-trimethylsilyl-ethyl)ester (1-Isocyano-2-methyl)-propyl-2-kohlensäuremethylester.

Weiter werden Verfahren zur Herstellung von Verbindungen der allgemeinen Formel I bereitgestellt:

Ein Verfahren ist dadurch gekennzeichnet, daß ein Isocyanoalkylalkoholatanion CN-CR₁R₂-CR₃R₄-O⁻ bzw. CN-CR₁R₂-CR₃R₄-CR₆R₇-O⁻ mit einem Halogenameisensäurederivat X-CO-Z-R₅ z.B. in einem aprotischen Lösungsmittel wie THF, Et₂O, DME oder CH₂Cl₂ zum entsprechenden Isocyanoalkylkohlensäurederivat der Formel I beispielhaft nach folgendem Schema umgesetzt wird.

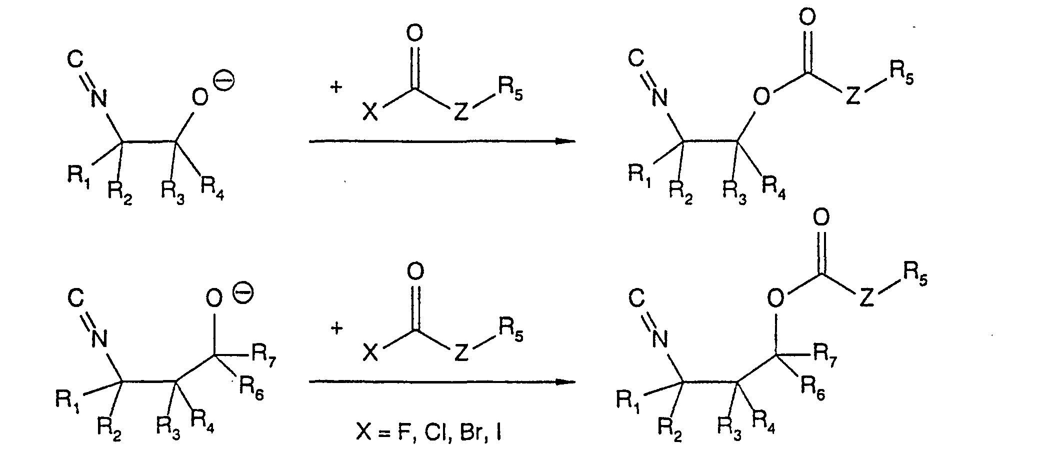

Die Isocyanoalkylalkoholatanionen können entweder aus den entsprechenden Isocyanoalkylalkoholen bzw. den cyclischen Imidoestern (2-Oxazolin bzw. 2-Oxazin) durch Umsetzung mit einer Base (wie z.B. n-BuLi), wie in der Literatur [D. Hoppe, *Angew. Chem.* **1974,** *86*, 878.] beschrieben, hergestellt werden.

Die 2-Oxazoline bzw. 2-Oxazine können aus den entsprechenden 2-Aminoethanolen bzw. 3-Aminopropanolen durch Umsetzung mit Ameisensäure [A.I. Meyers et al, *Tetrahedron* **1994,** *50,* 2297.] oder Dimethylformamiddimethylacetal [A.I. Meyers et al, *J. Org. Chem.* **1991,** *56,* 1961.] leicht hergestellt werden.

Die benötigten Isocyanide können entweder aus den entsprechenden Aminen durch Umsetzung mit Chloroform und starken Basen wie z.B. Alkalihydroxid (Carbylamin-Reaktion) [I. Ugi et al in: *"Isonitrile Chemistry*", I. Ugi (Hrsg.), Academic Press, New York, **1971**] oder aus den entsprechenden Formamiden durch Umsetzung mit POCl₃ oder Phosgen [I. Ugi et al, *Angew. Chem.* **1965,** 77, 492.] in Gegenwart von Stickstoffbasen hergestellt werden.

Ein weiteres Verfahren zur Herstellung von Verbindungen der allgemeinen Formel I ist dadurch gekennzeichnet, daß ein Formamidoalkylkohlensäurederivat OCH-NH-CR₁R₂-CR₃R₄-O-CO-Z-R₅ bzw. OCH-NH-CR₁R₂-CR₃R₄-CR₆R-₇-O-CO-Z-R₅ z. B. in einem aprotischen Lösungsmittel wie CH₂Cl₂ durch Wasserabspaltung zum entsprechenden Isocyanoalkylkohlen-säurederivat der Formel I beispielhaft nach folgendem Schema umgesetzt wird. 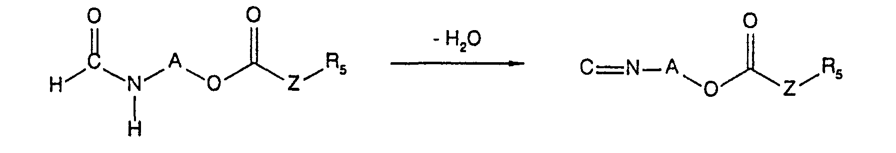 Die Wasserabspaltung kann z.B. durch Umsetzung mit Phosgen bzw. Di- oder Triphosgen oder Phosphoroxychlorid in Gegenwart von Stickstoffbasen geschehen. [I. Ugi, et al, *Angew. Chem.* **1965,** 77, 492.]

Die Formamidoalkylkohlensäurederivate können durch Umsetzung von Chlorameisensäurederivaten mit Formamidoalkylalkoholen erhalten werden, die wiederum aus den käuflich erhältlichen Aminoalkylalkoholen durch Umsetzung mit Ameisensäuremethylester hergestellt werden können.

Beide Verfahren eignen sich auch zur Herstellung von Isocyanoalkylkohlensäurederivaten, die über einen oder mehrere der Reste R₁ bis R₄, bzw. R₆ und R₇, sowie R₅ oder Z an eine feste Phase (Harz) gebunden sind, z.B. an Harze, die mit einer Carbonylfunktion, derivatisiert sind. Derartige Harze sind käuflich erhältlich. Bei dem Verfahren werden z.B. harzgebundene Chlorameisensäureester bzw. Isocyanide eingesetzt, die durch Derivatisierung von OH-funktionalisierten bzw. SH-funktionalisierten Harzen mit Phosgen [G. Skorna, I. Ugi, *Chem. Ber.* **1978,** *111*, 3965.] bzw. Isocyanoalkylmethylester hergestellt werden können. Auch die Wasserabspaltung von entsprechend eingesetzten harzgebundenen Formamiden kann problemlos durchgeführt werden. [Mjalli A.M.M. et al, *Tetrahedron Lett.* **1996,** *37,* 1149.]

Alle Verfahren eignen sich auch zur Herstellung von chiralen Isocyanoalkylkohlensäurederivaten.

### Isocyanid-Multikomponentenreaktionen

Unter einer Multikomponentenreaktion versteht man eine Reaktion, bei der das Produkt aus mindestens drei verschiedenen Edukten gebildet wird und wesentliche Teile dieser Edukte enthält.

Das charakteristische an Isocyanid-MCRs ist, daß das zweibindige C-Atom der Isocyanidfunktion im Verlauf dieser MCRs in einer intramolekularen Umlagerung irreversibel und unter Freisetzung von Energie zu einem vierbindigen C-Atom einer sekundären Amidfunktion umgewandelt wird. Dies stellt die thermodynamische Triebkraft der Isocyanid-MCRs dar. Diese Triebkraft führt dazu, daß diese intramolekulare Umlagerung unabhängig vom sterischen Anspruch der jeweiligen Substituenten erfolgt, so daß praktisch beliebige Substituenten eingesetzt werden können. Daraus ergibt sich eine enorme Bandbreite bezüglich der einsetzbaren Säure-, Carbonyl-, Isocyanid-, und gegebenenfalls der Aminkomponenten in den Isocyanid-MCRs. Aus diesem Grund werden die Isocyanid-MCRs in der Kombinatorischen Chemie, deren Ziel die Herstellung von vielen verschiedenen Molekülen in einer Eintopfsynthese ist, intensiv erforscht [F. Balkenhohl, *Angew. Chem.* **1996,** *108*, 2463.]. In der Literatur sind eine große Anzahl verschiedener Passerini- und Ugi-Produkte hergestellt und beschrieben worden [I. Ugi, S. Lohberger, R. Karl in: *Comprehensive Organic Synthesis: Selectivity for Synthetic Efficiency, Band II*, B. M. Trost, C.H. Heathcock, Pergamon Press, Oxford, **1991;** I. Ugi, *Angew. Chem.* **1962,** *74,* 9].

Mit den Isocyanid-MCRs können einfach und schnell komplizierte Moleküle in einer Eintopfsynthese aufgebaut werden. Die Vorteile gegenüber den üblichen mehrstufigen Herstellungsmethoden sind Zeitersparnis, Materialersparnis, höhere Ausbeuten bei weniger Nebenprodukten und dadurch ein geringerer Reinigungsaufwand. Der Nachteil der bisherigen Isocyanid-MCR-Produkte ist, daß die aus dem Isocyanid gebildete sekundäre Amidbindung nicht, nur bei bestimmten Systemen oder nur unter drastischen Bedingungen modifizierbar ist.

Die Isocyanid-MCRs können in einem Temperaturintervall von -90°C bis 90°C, vorzugsweise zwischen -40°C und 40°C, durchgeführt werden. Bei tiefen Temperaturen verlaufen die MCRs langsamer, bei zu hohen Temperaturen kann das Isocyanid zerstört werden.

Als Lösungsmittel eignen sich alle inerten Lösungsmittel, die mit den eingesetzten Komponenten nicht oder nur sehr langsam reagieren wie z.B. niedere Alkohole, CH₂Cl₂, Hexan, Trifluorethanol, THF, oder CH₃CN.

Die Reaktionszeiten können zwischen einigen Minuten und einigen Wochen schwanken.

Der Konzentrationsbereich der Komponenten kann zwischen 0.01 molar und 10 molar, vorzugsweise zwischen 0.1 und 2 molar sein. Ist die Konzentration zu gering, verlaufen die Isocyanid-MCRs nur sehr langsam, bei zu hohen Konzentrationen kann es zu Nebenreaktionen kommen. Die Komponenten werden vorzugsweise äquimolar eingesetzt. Es können aber auch einzelne Komponenten im Überschuß eingesetzt werden, da die entstehenden MCR-Produkte nicht weiter mit den eingesetzten Komponenten reagieren.

Erfindungsgemäß werden ferner sek.Amidoalkylkohlensäurederivate der Formeln III bis IX und Verfahren zur deren Herstellung offenbart.

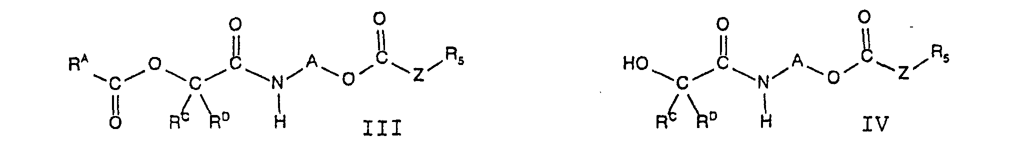

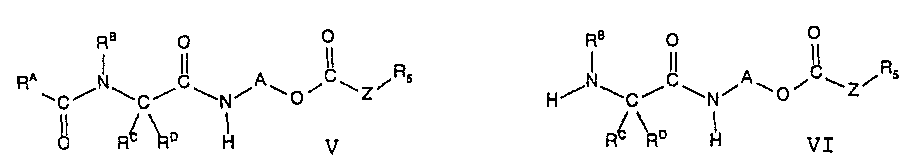

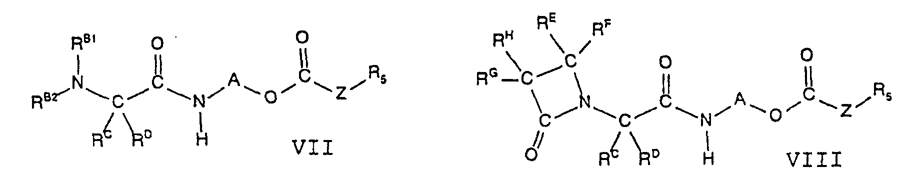

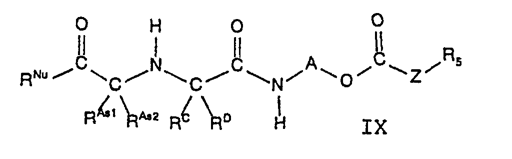

In den Formeln III bis IX sind die Reste wie folgt definiert:
Z, A und der Rest R₅, sind wie oben definiert;
die Reste R^{A}, R^{B}, R^{C}, R^{D}, R^{As1}, R^{As2}, R^{E}, R^{F}, R^{G}, R^{H}, R^{B1} und R^{B2} umfassen jeweils maximal 20 C-Atome und sind unabhängig voneinander H-Atome oder unsubstituierte Alkyl-, Alkenyl-, Alkinyl-, Alkaryl-, Aryl-, alicyclische Reste, heteroalicyclische oder Heteroarylreste mit bis zu 6 Heteroatomen (Heteroatome sind O, N, S) oder sind entsprechende Reste, die mit einer, zwei, drei oder mehr Halogen-, tert.Amino-, Nitro-, Cyano-, Carbonsäureester-, Carbonsäureamid-, Carbonsäureimid-, Alkyloxy-, Epoxy-, Bor-, Silyloxy-, Silyl-, Thio-, Sulfonsäureester-, Sulfensäureester-, Sulfinsäureester-, Sulfonamido-, Urethan-, oder Harnstoff-Funktion substituiert sind, oder R^{B} eine Gruppe der Formel OH, RR'N- (wobei R und R' unabhängig voneinander wie R^{A} definiert sein können, aber davon unabhängig sind) oder ein mit C₁-C₆ Alkyl bzw. C₁-C₆ Alkylcarbonyl O-geschützter 1-Aminocarbohydratrest ist; vorzugsweise umfassen diese Reste jeweils maximal 10 C-Atome und sind wie oben definiert.

R^{Nu} ist eine Gruppe der Formel HO-, R^{Alk}O- oder R^{Am1}R^{Am2}N- , wobei R^{Alk} ein Methyl-, Ethyl-, Propyl-, Butyl-, Allyl-, oder Benzylrest ist und R^{Am1} und R^{Am2} C₁-C₆ Alkylreste sind, bzw. R^{Am1} und R^{Am2} Bestandteile eines 5- oder 6-gliedrigen Cycloalkylringes, oder 6-gliedrigen Cycloalkylringes mit einem weiteren Heteroatom (N,O) sind.

Allgemein können die Reste R^{A}, R^{B}, R^{C}, R^{D}, R^{As1}, R^{As2}, R^{E}, R^{F}, R^{G}, R^{H}, R^{B1}, R^{B2}, R und R' mit allen funktionellen Gruppen substituiert sein, die nicht oder nur sehr langsam mit den funktionellen Gruppen der Isocyanid-MCR-Komponenten reagieren, vgl. z.B. [I. Ugi, S. Lohberger, R. Karl in: *Comprehensive Organic Synthesis: Selectivity for Synthetic Efficiency, Band II,* B. M. Trost, C.H. Heathcock, Pergamon Press, Oxford, **1991;** I. Ugi, *Angew. Chem.* **1962,** *74*, 9]. So können z.B. Aldehydketone in der U-4CR eingesetzt werden, wobei bei äquimolarem Einsatz aller Komponenten nur die Aldehydfunktion aufgrund ihrer höheren Reaktivität in der U-4CR reagiert.

### sek.Amidoalkylkohlensäurederivate der Formeln III und IV

In der Passerini-Reaktion [M. Passerini, *Gazz. Chim. Ital*., **1921,** 51 (11); I. Hagedorn, U. Eholzer, *Chem. Ber.* **1965,** *98,* 936.] werden durch den Einsatz einer Säurekomponente, einer Carbonylkomponente (Aldehyd bzw. Keton) und einer Isocyanidkomponente in einer Multikomponentenreaktion α-Acyloxyamide bzw. α-Hydroxyamide beispielhaft nach folgendem Schema aufgebaut.

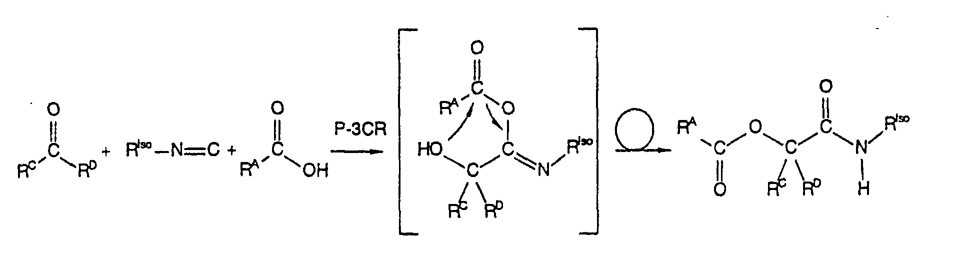

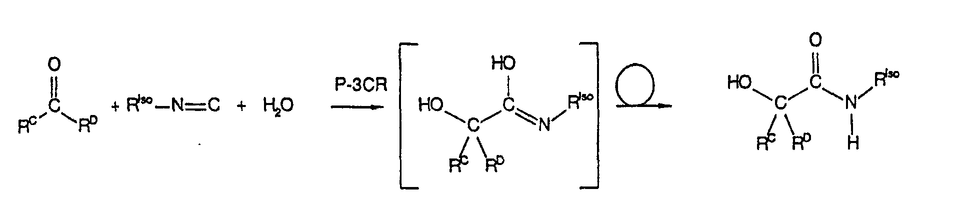

Wird in der P-3CR eine Carbonylkomponente R^{C}-CO-R^{D}, das Isocyanoalkylkohlensäurederivat der Formel I und eine Carbonsäure (R^{A}-COOH) als Säurekomponente zur Reaktion gebracht, entsteht das sek.Amidoalkylkohlensäurederivat III; mit Wasser als Säurekomponente entsteht das sek.Amidoalkylkohlensäurederivat IV.

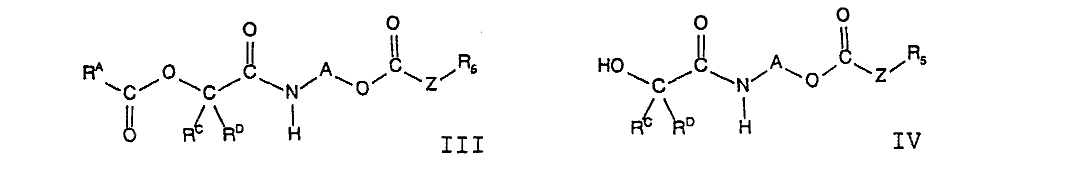

### Herstellung von sek.Amidoalkylkohlensäurederivaten der Formeln III und IV mittels P-3CR

Eine Carbonsäure R^{A}-COOH oder Wasser werden mit einer Carbonylkomponente R^{C}-CO-R^{D} und dem Isocyanoalkylkohlen-säurederivat der Formel I in einer P-3CR umgesetzt. Die Durchführung kann beispielsweise wie in der Literatur beschrieben erfolgen [M. Passerini, *Gazz. Chim. Ital.,* **1921,** 51 (11); I. Hagedorn, U. Eholzer, *Chem. Ber.* **1965,** *98*, 936.].

### sek.Amidoalkylkohlensäurederivate der Formeln V, VI und VII

In der U-4CR werden durch den Einsatz einer Säurekomponente z.B. Carbonsäure oder Wasser, einer Carbonylkomponente, einer Aminkomponente (z.B. primäres oder sekundäres Amin) und einer Isocyanidkomponente, α-Aminoacylamide oder α-Aminoamide beispielhaft nach folgendem Schema erhalten. 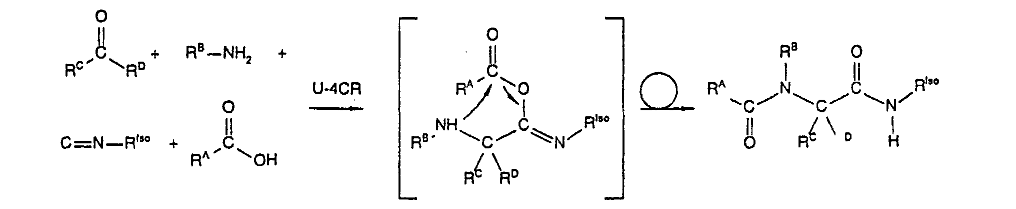 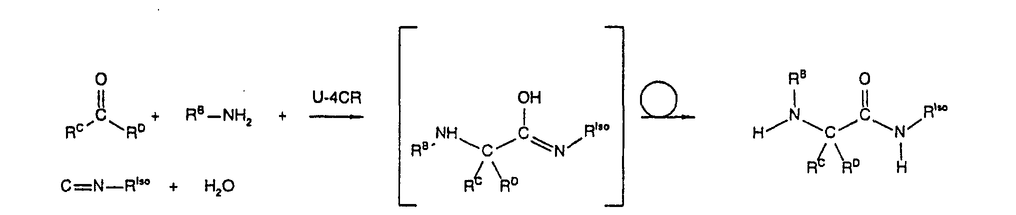 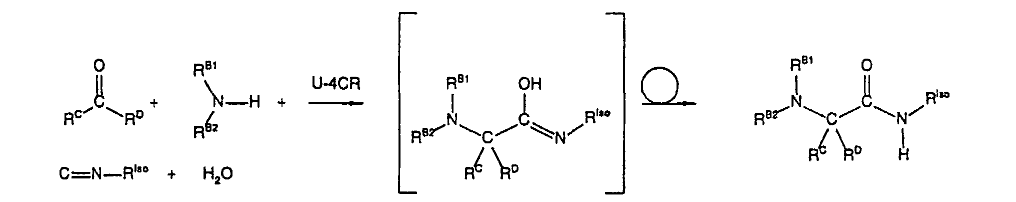 Werden in der U-4CR eine Carbonylkomponente R^{C}-CO-R^{D}, das Isocyanoalkylkohlensäurederivat der Formel I, eine Carbonsäure (R^{A}-COOH) und ein primäres Amin (R^{B}-NH₂) eingesetzt, entsteht das sek.Amidoalkylkohlensäurederivat V; mit Wasser als Säurekomponente und einem primären Amin (R^{B}-NH₂) entsteht das sek.Amidoalkylkohlensäurederivat VI. Werden in der U-4CR eine Carbonylkomponente R^{C}-CO-R^{D}, das Isocyanoalkylkohlensäure-derivat der Formel I , ein sekundäres Amin (R^{B1}-NH-R^{B2}) und Wasser als Säurekomponente eingesetzt, wird das sek.Amidoalkylkohlensäurederivat VII gebildet.

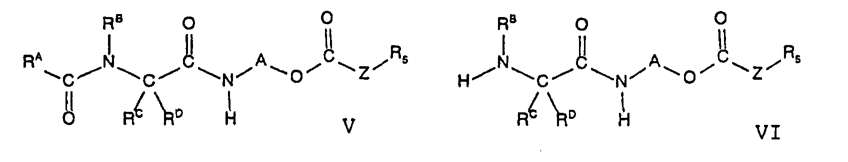

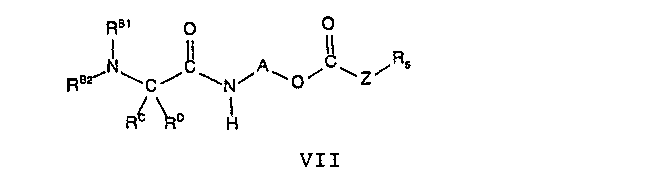

Als Aminkomponenten können Ammoniak, primäre Amine, O-acylierte bzw. O-alkylierte 1-Aminocarbohydrate, Hydroxylamine [NH₂OH] oder Hydrazine [H₂NNR(R')] und deren Derivate dienen.

Als Carbonylkomponenten eignen sich sowohl Aldehyde (auch sterisch anspruchsvolle), als auch Ketone (außer Diarylketone). Gelegentlich ist eine Vorkondensation des Imins nötig.

Aus den sek.Amidoalkylkohlensäurederivaten der Formeln V, VI und VII lassen sich z.B. N-Acyl-α-Aminosäurederivate, N-Acyl-N-alkyl-α-Aminosäurederivate, oder N,N'-Dialkyl-α-Aminosäurederivate herstellen.

### Herstellung der sek.Amidoalkylkohlensäurederivate der Formeln V, VI und VII mittels U-4CR

Eine Carbonsäure R^{A}-COOH oder Wasser wird mit einer Carbonylkomponente R^{C}-CO-R^{D}, einer Aminkomponente R^{B}-NH₂ oder R^{B1}-NH-R^{B2} und dem Isocyanoalkylkohlensäurederivat der Formel I in einer Ugi-4-Komponentenreaktion umgesetzt. Die Durchführung kann beispielsweise wie in der Literatur beschrieben erfolgen [I. Ugi et al, Chem. Ber. **1961**, *94*, 2802.].

### Monocyclische sek.Amidoalkylkohlensäurederivate der Formel VIII

Die Synthese von monocyclischen sek.Amidoalkylkohlensäure-derivaten der Formel VIII mittels U-4CR/β-AS stellt eine Variante der U-4CR dar, bei der sowohl die Amin-, als auch die Säurekomponente in einem einzigen Edukt, einer β-Aminosäure vorliegen. Die β-Aminosäure wird mit einer Carbonylkomponente und einer Isocyanidkomponente in einer U-4CR/β-AS beispielsweise nach folgendem Schema umgesetzt.

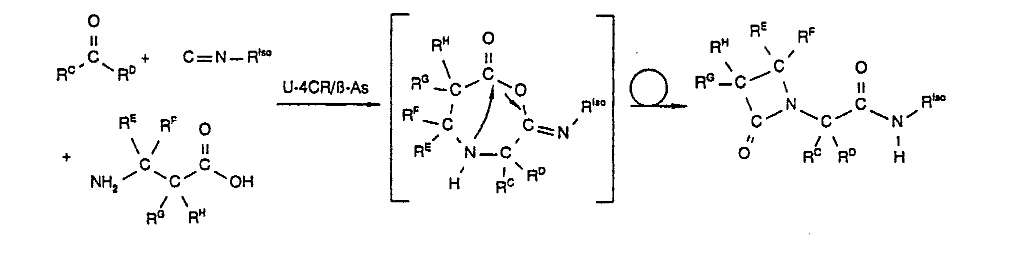

Hierbei bildet sich bei Verwendung einer Carbonylkomponente R^{C}-CO-R^{D}, des Isocyanoalkylkohlensäurederivates der Formel I und einer β-Aminosäure H₂N-CP^{F}R^{F}-CR^{G}R^{H}-COOH als Reaktionsprodukt das sek.Amidoalkylkohlensäurederivat VIII.

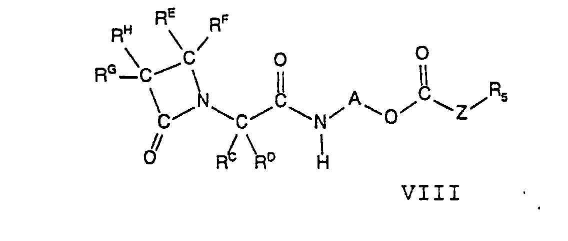

### Herstellung von monocyclischen sek.Amidoalkylkohlensäure-derivaten der Formel VIII mittels U-4CR/β-As

Eine β-Aminosäure H₂N-CR^{E}R^{F}-CR^{G}R^{H}-COOH wird mit einer Carbonylkomponente R^{C}-CO-R^{D} und dem Isocyanoalkylkohlensäure-derivat der Formel I in einer U-4CR/β-As umgesetzt. Die Durchführung kann beispielsweise wie in der Literatur beschrieben erfolgen [I. Ugi et al, *Tetrahedron* **1995,** *51,* 139].

### sek.Amidoalkylkohlensäurederivate der Formel IX

Die U-5C-4CR stellt eine wohlbekannte Variante der U-4CR dar, und dient zur Herstellung von 1,1'-Iminodicarbonsäurederivaten [W. Hörl, Dissertation, Technische Universität München, **1996.].** In der U-5C-4CR werden eine α-Aminosäure, eine Carbonyl-komponente, eine Isocyanidkomponente und ein Nukleophil, welches ein Alkohol, ein sekundäres Amin oder Wasser sein kann, beispielhaft nach folgendem Schema umgesetzt. 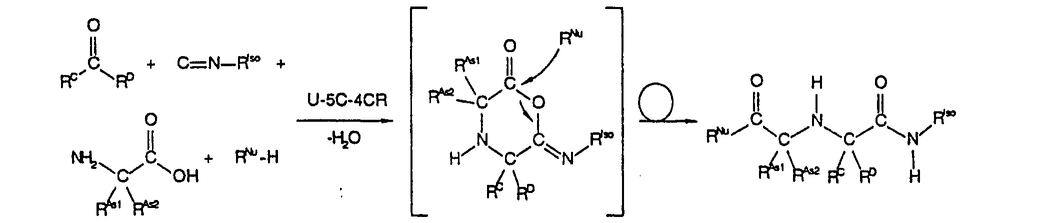 Ist R^{Nu}-H ein Alkohol oder Wasser, so wird es im Überschuß eingesetzt. In der U-5C-4CR bildet sich bei Verwendung eine α-Aminosäure H₂N-CR^{As1}R^{As2}-COOH, einer Carbonylkomponente R^{C}-CO-R^{D}, dem Isocyanoalkylkohlensäurederivat der Formel I und einem Nukleophil R^{Nu}-H das sek.Amidoalkylkohlensäurederivat IX.

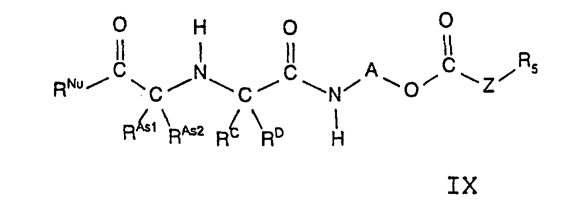

### Herstellung von sek.Amidoalkylkohlensäurederivaten der Formel IX mittels U-5C-4CR

Eine α-Aminosäure H₂N-CR^{As1}R^{As2}-COOH wird mit einer Carbonylkomponente R^{C}-CO-R^{D}, dem Isocyanoalkylkohlensäure-derivat der Formel I und einem Nukleophil R^{Nu}-H in einer Ugi-5-C-4CR umgesetzt. Die Durchführung kann beispielsweise wie in der Literatur beschrieben erfolgen [I. Ugi et al, *Tetrahedron.* **1996,** *52*, 11657].

Ist beispielsweise R^{Nu}-H ein Alkohol wird eine α-Aminosäure, eine Carbonylkomponente und das Isocyanoalkylkohlensäure-derivat I in einem Alkohol, der gleichzeitig Lösungsmittel und Nukleophil ist, gelöst bzw. suspendiert und gerührt. Nach Beendigung der Reaktion wird das Lösungsmittel entfernt und das Reaktionsprodukt, wenn nötig, gereinigt.

Ist beispielsweise R^{Nu}-H ein sekundäres Amin wird eine α-Aminosäure, eine Carbonylkomponente, das Isocyanoalkylkohlen-säurederivat I und ein sekundäres Amin in einem Lösungsmitel/Wasser-Gemisch wie z.B. einem THF/H₂O-Gemisch gelöst bzw. suspendiert und gerührt. Nach Beendigung der Reaktion wird das Lösungsmittel entfernt und das Reaktionsprodukt, wenn nötig, gereinigt.

Ist beispielsweise R^{Nu}-H Wasser wird eine α-Aminosäure, eine Carbonylkomponente und das Isocyanoalkylkohlensäurederivat I in Lösungsmitel/Wasser-Gemisch wie z.B. einem THF/H₂O-Gemisch gelöst bzw. suspendiert und gerührt. Nach Beendigung der Reaktion wird das Lösungsmittel entfernt und das Reaktionsprodukt, wenn nötig, gereinigt.

### Spaltung der sekundären Amidbindung der sek.Amidoalkylkohlensäurederivate der Formeln III bis IX

Ferner ist es möglich, die neuen sek.Amidoalkylkohlensäurederivate der Formeln III bis IX durch Umsetzung mit einer Base problemlos in α-Hydroxycarbonsäure- oder α-Aminocarbonsäurederivate zu überführen.

Durch Umsetzung der sek.Amidoalkylkohlensäurederivate mit einer Base wird das sek. Amidproton abstrahiert. Als Base eignen sich z.B. milde, nicht nukleophile Basen wie z.B. Kaliumtert.Butanolat, Alkalihydroxid, Alkalihydrid oder Stickstoffbasen wie Lithiumdiisopropylamin oder 1,5-Diazabicyclo[4.3.0]non-5-en beispielsweise in einem aprotischen Lösungsmittel wie z.B. THF, Et₂O, oder CH₂Cl₂. Die Umsetzung kann bei Temperaturen von -80°C bis 80°C erfolgen. Durch den anschließenden intramolekularen Ringschluß beispielhaft nach folgendem Schema werden primäre Spaltungsprodukte X erzeugt, die eine cyclische N-Acylurethan-Funktion aufweisen.

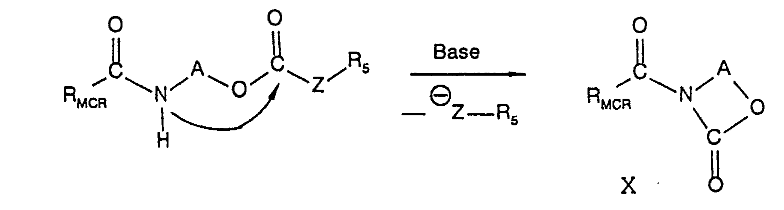

Der Rest R_{MCR} definiert die Strukturen, die durch die P-3CR-, die U-4CR-, die U-4CR/β-As- bzw. die U-5C-4CR aufgebaut werden. Bei dem Ringschluß wird Z-R₅ als Alkoholat- bzw. Thiolat-Anion abgespalten. Der weitere Reaktionsverlauf ist von der Struktur von R⁵ abhängig. Ist R⁵ ein Rest, der die Nukleophilie der Z-Funktion herabsetzt (z.B. ein elektronenziehender Rest, wie z.B. ein Phenylrest), so ist X isolierbar. Handelt es sich bei R⁵ um einen Rest, der die Nukleophilie der Z-Funktion erhöht (z.B. ein elektronen-schiebender Rest, wie z.B. ein Alkylrest), so ist X nicht isolierbar, sondern setzt sich in situ mit dem durch den intramolekularen Ringschluß gebildeten Alkoholat bzw. Thiolat zum entsprechenden Ester bzw. Thioester (R_{MCR}-CO-Z-R₅) beispielhaft nach folgendem Schema um.

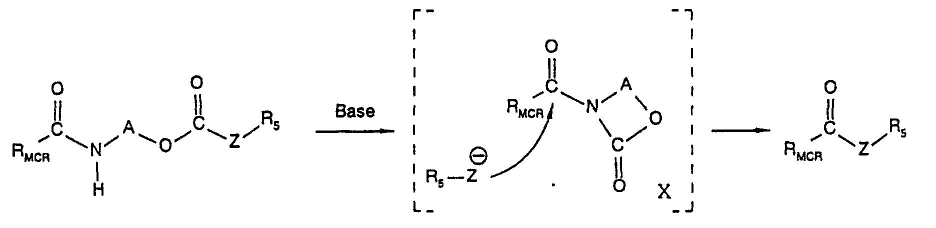

Auf diese Weise ist es möglich, die sek. Amidbindung der sek.Amidoalkylkohlensäurederivate der Formeln III bis IX direkt in den entsprechenden Ester zu überführen. Es können so z.B. durch die Wahl des entsprechenden Restes R⁵ in den Isocyanoalkylkohlensäurederivaten in einer Eintopfsynthese, die aus der Literatur bekannten Carboxylschutzgruppen [E. Haslam, Tetrahedron 1980, 36, 2409] direkt eingeführt werden.

Wie vorstehend ausgeführt ist X isolierbar, wenn es sich bei R⁵ um einen Rest wie z.B. einen phenylischen Rest handelt, der die Nukleophilie der Z-Funktion herabsetzt. Aufgrund seiner verringerten Nukleophilie, bedingt durch die aromatische Mesomerie, ist z.B. das Phenolat nicht in der Lage, X nukleophil anzugreifen und so den Phenylester zu bilden. Damit eröffnet sich die Möglichkeit, das entsprechende primäre Spaltungsprodukt X zu isolieren und anschließend mit einem Nukleophil wie einem Alkoholat, Thiolat, Hydroxyl- oder Amino-Anionen beispielhaft nach folgendem Schema umzusetzen, um die entsprechenden Ester, Thioester, Amide oder Carbonsäuren herzustellen.

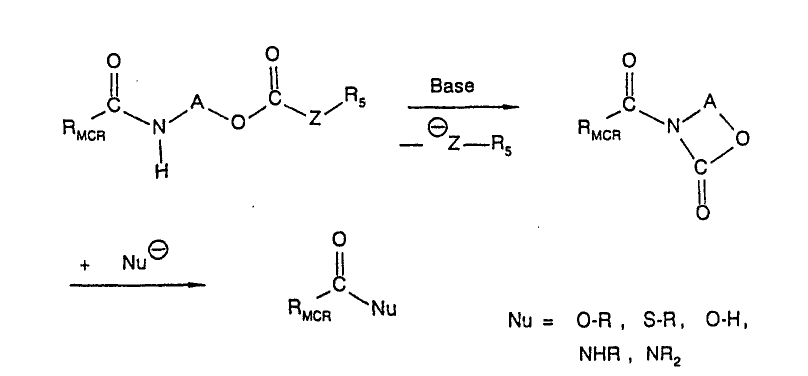

Da das erfindungsgemäße Verfahren zur Spaltung von sek. Amidbindungen auch bei milden basischen Bedingungen verläuft, kann die Spaltung der sek. Amidbindungen der sek.Amidoalkylkohlensäurederivate unter Erhalt des Acetidinon-Ringes erfolgen.

Zusammenfassend läßt sich sagen, daß alle mit den neuen Isocyanoalkylkohlensäurederivaten herstellbaren sek.Amidoalkylkohlensäurederivate zu den entsprechenden α-Hydroxycarbonsäure- oder α-Aminocarbonsäurederivaten umgesetzt werden können, da die Spaltungsmethode unabhängig von der Struktur des restlichen Moleküls ist.

### Spaltungsvorschrift der sek.Amidoalkylkohlensäurederivate

Die sek.Amidoalkylkohlensäurederivate der Formeln III bis IX werden mit einer Base umgesetzt. Beispielsweise wird das sek.Amidoalkylkohlensäurederivat in einem inerten, aprotischen Lösungsmittel wie z.B. THF, Et₂O oder CH₂Cl₂ gelöst und eine Base wie z.B. Kalium-tert.Butanolat, zugegeben. Nach Beendigung der Reaktion wird das Lösungsmittel entfernt, und das Reaktionsprodukt, das je nach Struktur des Restes R₅ und der Art der eingesetzten Base das primäre Spaltungsprodukt X, ein α-Hydroxycarbonsäure- oder α-Aminocarbonsäurederivat sein kann, gereinigt.

### Beispiele

### Beispiel 1: Herstellung von (2-Isocyano-2-methyl)-propyl-1-kohlensäureallylester aus 4,4-Dimethyl-2-oxazolin

0.1 mol 4,4-Dimethyl-2-oxazolin wird in 200 ml absolutem THF gelöst und bei -78°C langsam 37 ml einer 2.7 M n-BuLi-Lösung zugetropft. Nach einer Stunde Rühren bei -78°C gibt man langsam 0.1 mol Chlorameisensäureallylester zu, rührt noch 1 Stunde bei -78°C und läßt dann die Temperatur der Reaktionsmischung langsam auf 25 °C steigen. Nach einer Stunde Rühren bei 25 °C wird das Lösungsmittel abdestilliert, der Rückstand in Methylenchlorid aufgenommen und einmal mit Wasser und anschließend mit gesättigter Kochsalzlösung gewaschen. Die organische Phase wird über Natriumsulfat getrocknet und das Lösungsmittel abgezogen. Man erhält (2-Isocyano-2-methyl)-propyl-1-kohlensäureallylester in 80%iger Ausbeute.

### Beispiel 2: Herstellung von (1-Isocyano-2-methyl)-propyl-2-kohlensäurebenzylester aus Methylisocyanid, Aceton und Chlorameisensäurebenzylester

0.1 mol Methylisocyanid wird in 200 ml absolutem THF vorgelegt und bei -78°C langsam 37 ml einer 2.7 M n-BuLi-Lösung zugetropft. Man rührt 1 Stunde bei -78°C und gibt anschließend langsam 0.1 mol absolutes Aceton zu. Nach 1 Stunde Rühren bei -78°C gibt man langsam 0.1 mol Chlorameisensäurebenzylester zu, rührt noch 1 Stunde bei -78°C und läßt dann die Temperatur der Reaktionsmischung langsam auf 25 °C steigen. Nach einer Stunde Rühren bei 25 °C wird das Lösungsmittel abdestilliert, der Rückstand in Methylenchlorid aufgenommen und einmal mit Wasser und anschließend mit gesättigter Kochsalzlösung gewaschen. Die organische Phase wird über Natriumsulfat getrocknet und das Lösungsmittel abgezogen. Man erhält (1-Isocyano-2-methyl)-propyl-2-kohlensäurebenzylester in 80%iger Ausbeute.

### Beispiel 3: Herstellung von (2-Isocyano-2-methyl)-propyl-1-kohlensäuremethylester durch Wasserabspaltung aus (2-Formamido-2-methyl)-propyl-1-kohlensäuremethylester

0.1 mol (2-Formamido-2-methyl)-propyl-1-kohlensäuremethylester und 0.3 mol Triethylamin werden in 300 ml absolutem Methylenchlorid gelöst und bei -20°C 0.033 mol Triphosgen portionsweise zugegeben. Danach rührt man 2 Stunden bei 0°C und anschließend 1 Stunde bei 25°C. Dann gibt man die Reaktionsmischung zu einer 10%igen Natriumhydrogencarbonat-Eiswasserlösung und extrahiert die wäßrige Phase dreimal mit Methylenchlorid. Die organische Phase wird über Natriumsulfat getrocknet, das Lösungsmittel abgezogen. Das Rohprodukt wird durch Säulenchromatographie über basischen Al₂O₃ gereinigt und man erhält (2-Isocyano-2-methyl)-propyl-1-kohlensäure-methylester in 80%iger Ausbeute.

### Beispiel 4: Herstellung von

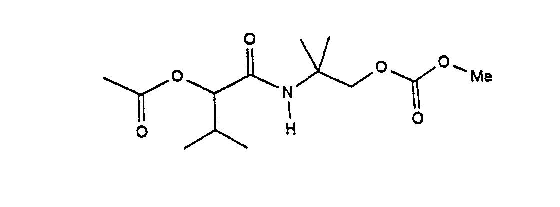

### aus Essigsäure, Isobutyraldehyd und (2 -Isocyano-2-methyl)-propyl-1-kohlensäuremethylester mittels P-3CR

Jeweils 10 mmol Essigsäure, Isobutyraldehyd und (2-Isocyano-2-methyl)-propyl-1-kohlensäuremethylester werden in 100 ml THF bei 25°C gerührt. Nach Beendigung der Reaktion wird das Lösungsmittel entfernt.
Aufarbeitung:
Der Rückstand wird in Methylenchlorid aufgenommen, mit Wasser gewaschen, die organische Phase über Magnesiumsulfat getrocknet und das Lösungsmittel abgezogen. In vielen Fällen liegt das Reaktionsprodukt nach dieser Aufarbeitungsprozedur als Reinstoff vor. Wenn nötig wird das Reaktionsprodukt durch Säulenchromatographie gereinigt.

### Beispiel 5: Herstellung von

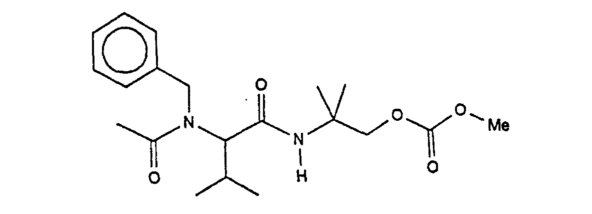

### aus Essigsäure, Benzylamin, Isobutyraldehyd und (2-Isocyano-2-methyl)-propyl-1-kohlensäuremethylester mittels U-4CR

Jeweils 10 mmol Benzylamin und Isobutyraldehyd werden in 100 ml Methanol vorgelegt und 1 Stunde bei 25°C unter Rühren über Molsieb vorkondensiert. Danach werden jeweils 10 mmol Essigsäure und (2-Isocyano-2-methyl)-propylkohlensäuremethylester zugegeben und gerührt. Nach Beendigung der Reaktion wird über Cellite filtriert und Lösungsmittel entfernt. Die Aufarbeitung erfolgt analog Beispiel 4.

### Beispiel 6: Herstellung von

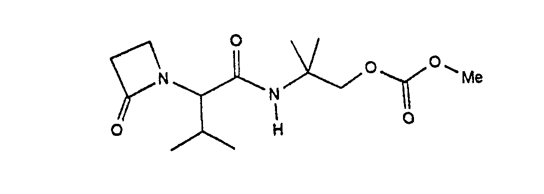

### aus β-Alanin, Isobutyraldehyd und (2-Isocyano-2-methyl)-propyl-1-kohlensäuremethylester mittels der U-4CR/β-AS

Jeweils 10 mmol β-Alanin und Isobutyraldehyd werden in Trifluorethanol vorgelegt und unter Verwendung von Molsieb 24 Stunden bei 25°C vorkondensiert. Danach wird 10 mmol (2-Isocyano-2-methyl)-propyl-1-kohlensäuremethylester zugefügt und gerührt. Nach Beendigung der Reaktion wird über Cellite filtriert und das Lösungsmittel entfernt. Die Aufarbeitung erfolgt analog Beispiel 4.

### Beispiel 7: Herstellung von

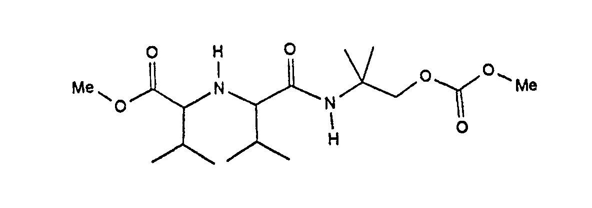

### aus Valin, Isobutyraldehyd und (2-Isocyano-2-methyl)-propyl-1-kohlensäuremethylester mittels U-5C-4CR mit Methanol als nukleophiler Komponente

10 mmol Valin werden in 100 ml Methanol suspendiert und jeweils 10 mmol Isobutyraldehyd und (2-Isocyano-2-methyl)-propyl-1-kohlensäuremethylester bei 25°C zugegeben und gerührt. Nach Beendigung der Reaktion wird das Lösungsmittel entfernt. Die Aufarbeitung erfolgt analog Beispiel 4.

### Beispiel 8: Herstellung von

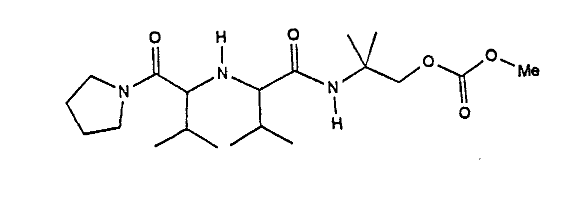

### aus Valin, Isobutyraldehyd und (2-Isocyano-2-methyl)-propyl-1-kohlensäuremethylester mit der U-5C-4CR mit Pyrrolidin als nukleophiler Komponente

10 mmol Valin werden in 100 ml Tetrahydrofuran suspendiert und bei 25°C jeweils 10 mmol Isobutyraldehyd, (2-Isocyano-2-methyl)-propyl-1-kohlensäuremethylester, Pyrrolidin und anschließend 20 ml Wasser zugegeben und gerührt. Nach Beendigung der Reaktion wird das Lösungsmittel entfernt. Die Aufarbeitung erfolgt analog Beispiel 4.

### Beispiel 9: Herstellung von

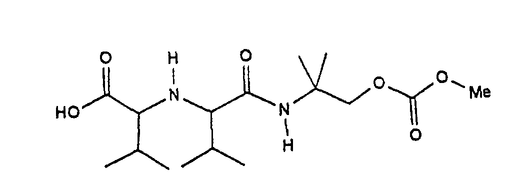

### aus Valin, Isobutyraldehyd und (2-Isocyano-2-methyl)-propyl-1-kohlensäuremethylester mit der U-5C-4CR mit Wasser als nukleophiler Komponente

10 mmol Valin werden in 100 ml eines 1:1 Gemisches Tetrahydrofuran/Wasser suspendiert und bei 25°C jeweils 10 mmol Isobutyraldehyd und (2-Isocyano-2-methyl)-propyl-1-kohlensäuremethylester zugegeben und gerührt. Nach Beendigung der Reaktion wird das Lösungsmittel entfernt. Die Aufarbeitung erfolgt analog Beispiel 4.

### Beispiel 10: Spaltung von

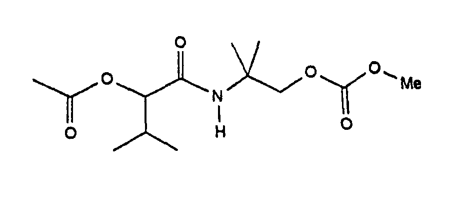

10 mmol der vorstehenden Verbindung werden in 100 ml absolutem THF gelöst. Anschließend wird eine Lösung aus 10 mmol Kaliumtert.Butanolat in absolutem THF bei 0°C zugetropft und gerührt. Nach Beendigung der Reaktion wird das Reaktionsgemisch über eine Kieselgelsäule filtriert. Das Lösungsmittel wird entfernt und die Spaltungsprodukte durch Säulenchromatographie getrennt und gereinigt.
Man erhält 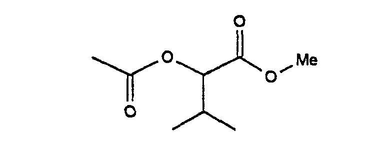 in 80%-iger Aubeute.

### Beispiel 11: Spaltung von

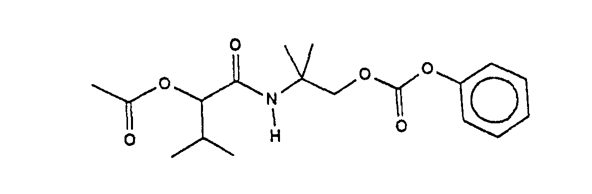

10 mmol der vorstehenden Verbindung werden in 100 ml absolutem THF gelöst. Anschließend wird eine Lösung aus 10 mmol Kaliumtert.Butanolat in absolutem THF bei 0°C zugetropft und gerührt. Nach Beendigung der Reaktion wird das Reaktionsgemisch über eine Kieselgelsäule filtriert. Das Lösungsmittel wird entfernt und die Spaltungsprodukte durch Säulenchromatographie getrennt und gereinigt. Man erhält 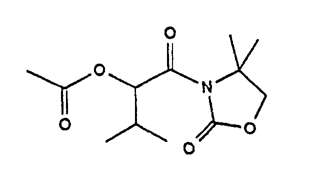 in 80%-iger Ausbeute.

## Patentansprüche

1. Verbindungen der allgemeinen Formel I 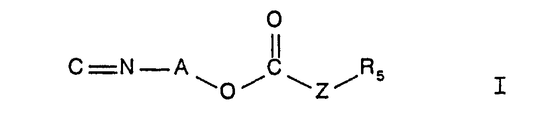 worin A eine Gruppe der Formel 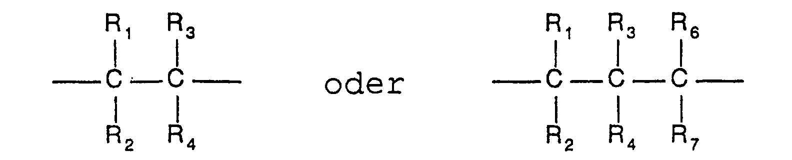 ist,
Z ein O-, oder S-Atom ist, und
die Reste R₁ bis R₄, bzw. R₆ und R₇ jeweils maximal 20 C-Atome umfassen und unabhängig voneinander H-Atome, substituierte oder unsubstituierte Alkyl-, Alkenyl-, Alkinyl-, Alkaryl-, Aryl-, alicyclische Reste, heteroalicyclische oder Heteroarylreste mit bis zu 4 Heteroatomen [Heteroatome sind O, N, S] sind, und optional jeweils zwei der Reste R₁ bis R₄ und gegebenenfalls R₆ und R₇, die durch bis zu zwei Kohlenstoffatome voneinander getrennt sind, Bestandteile eines gemeinsamen Ringsystems sind, wobei das Ringsystem einen substituierten oder unsubstituierten alicyclischen Monocyclus (5-8 Ringatome), einen heteroalicyclischen Monocyclus (5-8 Ringatome mit bis zu 2 Heteroatomen [O, N, S]), einen alicyclischen Bicyclus (7-14 Ringatome), oder einen heteroalicyclischen Bicyclus (7-14 Ringatome mit bis zu 4 Heteroatomen [O, N, S]) umfaßt, und
R₅ maximal 20 C-Atome umfaßt und ein substituierter oder unsubstituierter Alkyl-, Alkenyl-, Alkinyl-, Allyl,- Alkaryl-, Aryl, alicyclischer Rest, heteroalicyclischer oder Heteroarylrest mit bis zu 4 Heteroatomen (Heteroatome sind O, N, S) ist.

2. Verbindungen nach Anspruch 1, wobei die Substituenten von R₁ bis R₇ eine, zwei, drei oder mehrere Halogen-, tert.Amino-, Nitro-, Cyano-, Ether-, Silyloxyalkyl-, Thioether-, Carbonsäureester-, tert.Carbonsäureamid-, Carbonsäureimid-, Silyl-, Sulfonsäureester-, Sulfensäureester-, Sulfinsäureester-, tert.Sulfonamido-Funktionen sind.

3. Verbindungen nach einem der Ansprüche 1 oder 2, **dadurch gekennzeichnet, daß** A eine Gruppe der Formel -C(CH₃)₂-CH₂- ist.

4. Verbindungen nach einem der Ansprüche 1 bis 3, nämlich:
(2-Isocyano-2-methyl)-propyl-1-kohlensäureallylester, (2-Isocyano-2-methyl)-propyl-1-kohlensäurebenzylester, (2-Isocyano-2-methyl)-propyl-1-kohlensäure-(4-nitrobenzyl)ester, (2-Isocyano-2-methyl)-propyl-1-kohlensäureethylester, (2-Isocyano-2-methyl)-propyl-1-kohlensäurephenylester, (2-Isocyano-2-methyl)-propyl-2-kohlensäure-(4-nitrophenyl)ester, (2-Isocyano-2-methyl)-propyl-1-kohlensäurevinylester, (2-Isocyano-2-methyl)-propyl-2-kohlensäure-(2,2,2-trichlorethyl)-ester, (2-Isocyano-2-methyl)-propyl-1-kohlensäure-(2-trimethylsilyl-ethyl)ester, (2-Isocyano-2-methyl)-propyl-1-kohlensäuremethylester, (1-Isocyano-2-methyl)-propyl-2-kohlensäureallylester, (1-Isocyano-2-methyl)-propyl-2-kohlensäurebenzylester, (1-Isocyano-2-methyl)-propyl-2-kohlensäure-(4-nitrobenzyl)ester, (1-Isocyano-2-methyl)-propyl-2-kohlensäureethylester, (1-Isocyano-2-methyl)-propyl-2-kohlensäurephenylester, (1-Isocyano-2-methyl)-propyl-2-kohlensäure-(4-nitrophenyl)ester, (1-Isocyano-2-methyl)-propyl-2-kohlensäurevinylester, (1-Isocyano-2-methyl)-propyl-2-kohlensäure-(2,2,2-trichlorethyl)-ester, (1-Isocyano-2-methyl)-propyl-2-kohlensäure-(2-trimethylsilyl-ethyl)ester, (1-Isocyano-2-methyl)-propyl-2-kohlensäuremethylester.

5. Verbindungen der Formeln III bis IX, 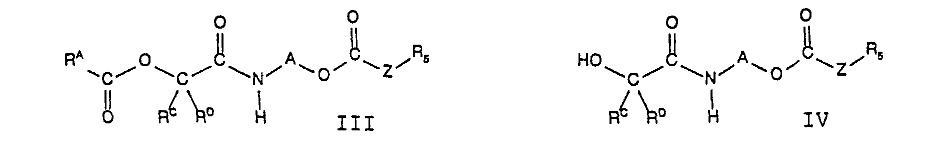 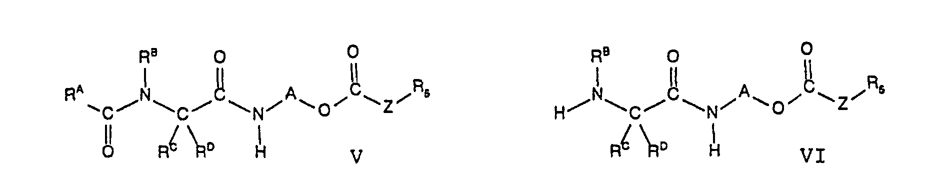 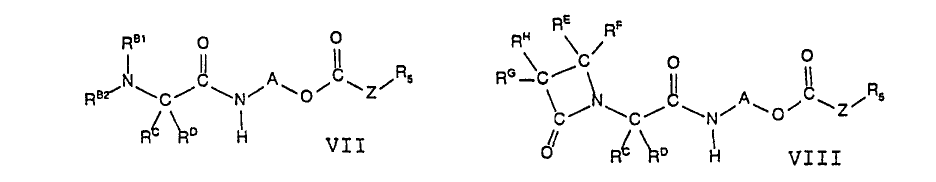 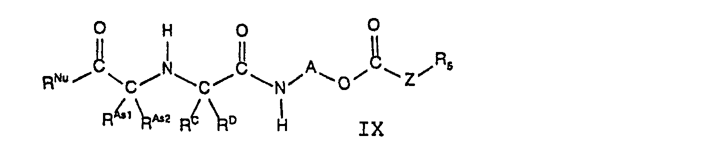 **dadurch gekennzeichnet, daß**
Z, A und der Rest R₅ wie in einem der Ansprüche 1 oder 2 definiert sind,
die Reste R^{A}, R^{B}, R^{C}, R^{D}, R^{As1}, R^{As2}, R^{E}, R^{F}, R^{G}, R^{H}, R^{B1} und R^{B2} jeweils maximal 20 C-Atome umfassen und unabhängig voneinander H-Atome oder unsubstituierte Alkyl-, Alkenyl-, Alkinyl-, Alkaryl, Aryl-, alicyclische Reste, heteroalicyclische oder Heteroarylreste mit bis zu 6 Heteroatomen (Heteroatome sind O, N, S) oder entsprechende Reste darstellen, die mit einer, zwei oder mehr Halogen-, tert.Amino-, Nitro-, Cyano-, Carbonsäureester-, Carbonsäureamid-, Carbonsäureimid-, Alkyloxy-, Epoxy-, Bor-, Silyloxy-, Silyl-, Thio-, Sulfonsäureester-, Sulfensäureester-, Sulfinsäureester-, Sulfonamido-, Urethan- oder Harnstoff-Funktion substituiert sind, oder R^{B} eine Gruppe der Formel OH, RR'N- (wobei R und R' unabhängig voneinander wie R^{A} definiert sind, aber von R^{A} unabhängig sind) oder ein mit C₁-C₆ Alkyl bzw. C₁-C₆ Alkylcarbonyl O-geschützter 1-Aminocarbohydratrest ist, und
R^{Nu} eine Gruppe der Formel HO-, R^{Alk}O- oder R^{Am1}R^{Am2}N- ist, wobei R^{Alk} ein Methyl-, Ethyl-, Propyl-, Butyl-, Allyl-, oder Benzylrest ist, R^{Am1} und R^{Am2} C₁-C₆ Alkylreste sind, bzw. R^{Am1} und R^{Am2} Bestandteile eines 5- oder 6-gliedrigen Cycloalkylringes, oder 6-gliedrigen Cycloalkylringes mit einem weiteren Heteroatom (N,O) sind.

6. Verbindungen nach einem der Ansprüche 1, 2 oder 5, **dadurch gekennzeichnet, daß** sie über einen oder mehrere der Reste R₁ bis R₄, bzw. R₆ und R₇, sowie R₅ oder Z an eine feste Phase gebunden sind.

7. Verfahren zur Herstellung von Verbindungen der Formeln III und IV nach Anspruch 5 oder 6, **dadurch gekennzeichnet, daß** eine Carbonsäure R^{A}-COOH oder Wasser mit einer Carbonylkomponente R^{C}-CO-R^{D} und dem Isocyanoalkylkohlensäurederivat der Formel I in einer Passerini-3-Komponentenreaktion umgesetzt wird.

8. Verfahren zur Herstellung von Verbindungen der Formeln V, VI, und VII nach Anspruch 5 oder 6, **dadurch gekennzeichnet, daß** eine Carbonsäure R^{A}-COOH oder Wasser mit einer Carbonylkomponente R^{C}-CO-R^{D}, einer Aminkomponente R^{B}-NH₂ oder R^{B1}-NH-R^{B2} und dem Isocyanoalkylkohlensäurederivat der Formel I in einer Ugi-4-Komponentenreaktion umgesetzt wird.

9. Verfahren zur Herstellung von Verbindungen der Formel VIII nach Anspruch 5 oder 6, **dadurch gekennzeichnet, daß** eine β-Aminosäure H₂N-CR^{E}R^{F}-CR^{G}R^{H}-COOH mit einer Carbonylkomponente R^{C}-CO-R^{D} und dem Isocyanoalkylkohlensäurederivat der Formel I in einer Ugi-4-Komponentenreaktion mit β-Aminosäuren umgesetzt wird.

10. Verfahren zur Herstellung von Verbindungen der Formel IX nach Anspruch 5 oder 6, **dadurch gekennzeichnet, daß** eine α-Aminosäure H₂N-CR^{As1}R^{As2}-COOH mit einer Carbonylkomponente R^{C}-CO-R^{D}, dem Isocyanoalkylkohlensäurederivat der Formel I und einem Nukleophil R^{Nu}-H in einer Ugi-5-Zentren-4-Komponentenreaktion umgesetzt wird.

## Claims

1. Compounds of the general formula I 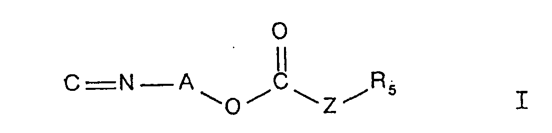 wherein A is a group of the formula 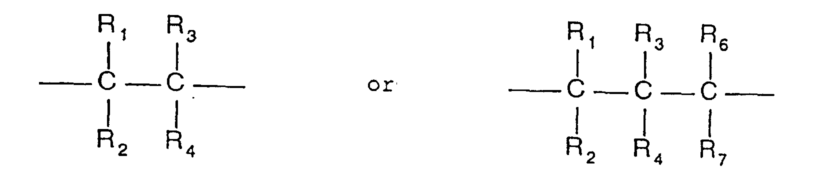 Z is an O or S atom, and
the radicals R₁ to R₄ and, when present, R₆ and R₇ each have a maximum of 20 carbon atoms and are independently of one another H atoms, substituted or unsubstituted alkyl, alkenyl, alkynyl, alkaryl, aryl, alicyclic radicals, heteroalicyclic or heteroaryl radicals having up to 4 hetero atoms [hetero atoms are O, N, S] , and optionally two of the radicals R₁ to R₄ and, when present, R₆ and R₇ that are separated from one another by up to two carbon atoms are constituents of a common ring system, the ring system comprising a substituted or unsubstituted alicyclic monocycle (5 to 8 ring atoms), a heteroalicyclic monocycle (5 to 8 ring atoms with up to 2 hetero atoms [O, N, S]), an alicyclic bicycle (7 to 14 ring atoms) or a heteroalicyclic bicycle (7 to 14 ring atoms with up to 4 hetero atoms [O, N, S]), and
R₅ has a maximum of 20 carbon atoms and is a substituted or unsubstituted alkyl, alkenyl, alkynyl, allyl, alkaryl, aryl, alicyclic radical, heteroalicyclic or heteroaryl radical having up to 4 hetero atoms (hetero atoms are O, N, S).

2. Compounds according to claim 1, wherein the substituents of R₁ to R₇ are one, two, three or more halogen, tert-amino, nitro, cyano, ether, silyloxyalkyl, thioether, carboxylic acid ester, tert-carboxylic acid amide, carboxylic acid imide, silyl, sulfonic acid ester, sulfenic acid ester, sulfinic acid ester or tert-sulfonamido functions.

3. Compounds according to claim 1 or 2, **characterised in that** A is a group of the formula -C(CH₃)₂-CH₂-.

4. Compounds according to any one of claims 1 to 3, namely:
(2-isocyano-2-methyl)-propyl-1-carbonic acid allyl ester,
(2-isocyano-2-methyl)-propyl-1-carbonic acid benzyl ester,
(2-isocyano-2-methyl)-propyl-1-carbonic acid (4-nitrobenzyl) ester,
(2-isocyano-2-methyl)-propyl-1-carbonic acid ethyl ester,
(2-isocyano-2-methyl)-propyl-1-carbonic acid phenyl ester,
(2-isocyano-2-methyl)-propyl-1-carbonic acid (4-nitrophenyl) ester,
(2-isocyano-2-methyl)-propyl-1-carbonic acid vinyl ester,
(2-isocyano-2-methyl)-propyl-1-carbonic acid (2,2,2-trichloroethyl) ester,
(2-isocyano-2-methyl)-propyl-1-carbonic acid (2-trimethylsilyl-ethyl) ester,
(2-isocyano-2-methyl)-propyl-1-carbonic acid methyl ester,
(1-isocyano-2-methyl)-propyl-2-carbonic acid allyl ester,
(1-isocyano-2-methyl)-propyl-2-carbonic acid benzyl ester,
(1-isocyano-2-methyl)-propyl-2-carbonic acid (4-nitrobenzyl) ester,
(1-isocyano-2-methyl)-propyl-2-carbonic acid ethyl ester,
(1-isocyano-2-methyl)-propyl-2-carbonic acid phenyl ester,
(1-isocyano-2-methyl)-propyl-2-carbonic acid (4-nitrophenyl) ester,
(1-isocyano-2-methyl)-propyl-2-carbonic acid vinyl ester,
(1-isocyano-2-methyl)-propyl-2-carbonic acid (2,2,2-trichloroethyl) ester,
(1-isocyano-2-methyl)-propyl-2-carbonic acid (2-trimethylsilyl-ethyl) ester,
(1-isocyano-2-methyl)-propyl-2-carbonic acid methyl ester.

5. Compounds of the formulae III to IX 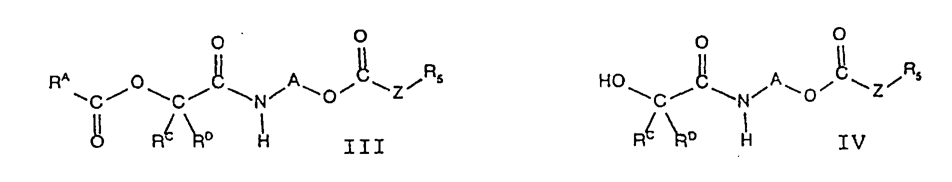 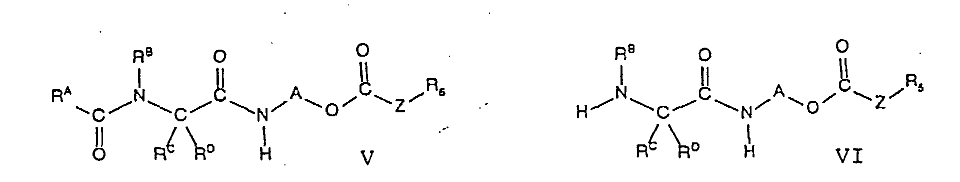 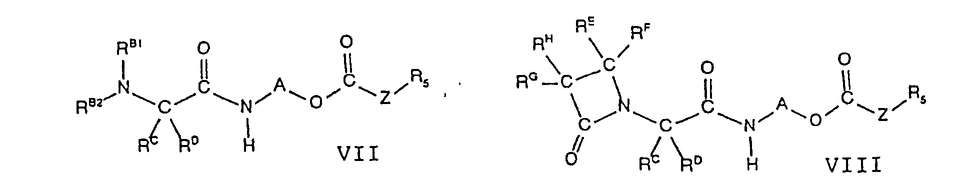 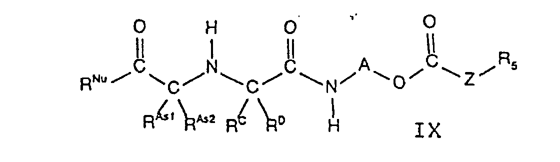 **characterised in that**
Z, A and the radical R₅ are as defined in claim 1 or 2, the radicals R^{A}, R^{B}, R^{C}, R^{D}, R^{As1}, R^{As2}, R^{E}, R^{F}, R^{G}, R^{H}, R^{B1} and R^{B2} each have a maximum of 20 carbon atoms and are independently of one another H atoms or unsubstituted alkyl, alkenyl, alkynyl, alkaryl, aryl, alicyclic radicals, heteroalicyclic or heteroaryl radicals having up to 6 hetero atoms (hetero atoms are O, N, S) or are corresponding radicals that are substituted by one, two or more halogen, tert-amino, nitro, cyano, carboxylic acid ester, carboxylic acid amide, carboxylic acid imide, alkyloxy, epoxy, boron, silyloxy, silyl, thio, sulfonic acid ester, sulfenic acid ester, sulfinic acid ester, sulfonamido, urethane or urea functions, or R^{B} is a group of the formula OH, RR'N- (wherein R and R' each independently of the other have the same definitions as R^{A} but are independent of R^{A}) or is a 1-aminocarbohydrate radical O-protected by C₁-C₆ alkyl or by C₁-C₆ alkylcarbonyl; and
R^{Nu} is a group of the formula HO-, R^{Alk}O- or R^{Am1}R^{Am2}N-, wherein R^{Alk} is a methyl, ethyl, propyl, butyl, allyl or benzyl radical and R^{Am1} and R^{Am2} are C₁-C₆ alkyl radicals, or R^{Am1} and R^{Am2} are constituents of a 5- or 6-membered cycloalkyl ring or 6-membered cycloalkyl ring having a further hetero atom (N, O).

6. Compounds according to any one of claims 1, 2 and 5, **characterised in that** they are bonded to a solid phase via one or more of the radicals R₁ to R₄ and, when present, R₆ and R₇, also R₅ or Z.

7. Process for the preparation of compounds of the formulae III and IV according to claim 5 or 6, **characterised in that** a carboxylic acid R^{A}-COOH or water is reacted with a carbonyl component R^{C}-CO-R^{D} and the isocyanoalkylcarbonic acid derivative of the formula I in a Passerini 3-component reaction.

8. Process for the preparation of compounds of the formulae V, VI and VII according to claim 5 or 6, **characterised in that** a carboxylic acid R^{A}-COOH or water is reacted with a carbonyl component R^{C}-CO-R^{D}, an amine component R^{B}-NH₂ or R^{B1}-NH-R^{B2} and the isocyanoalkylcarbonic acid derivative of the formula I in an Ugi 4-component reaction.

9. Process for the preparation of compounds of the formula VIII according to claim 5 or 6, **characterised in that** a β-amino acid H₂N-CR^{E}R^{F}-CR^{G}R^{H}-COOH is reacted with a carbonyl component R^{C}-CO-R^{D} and the isocyanoalkylcarbonic acid derivative of the formula I in an Ugi 4-component reaction with β-amino acids.

10. Process for the preparation of compounds of the formula IX according to claim 5 or 6, **characterised in that** an α-amino acid H₂N-CR^{As1}R^{As2}-COOH is reacted with a carbonyl component R^{C}-CO-R^{D}, the isocyanoalkylcarbonic acid derivative of the formula I and a nucleophile R^{Nu}-H in an Ugi 5-centre 4-component reaction.

## Revendications

1. Composés de formule générale I : 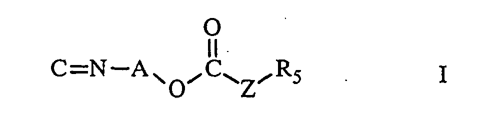 dans laquelle A représente un groupe de formule 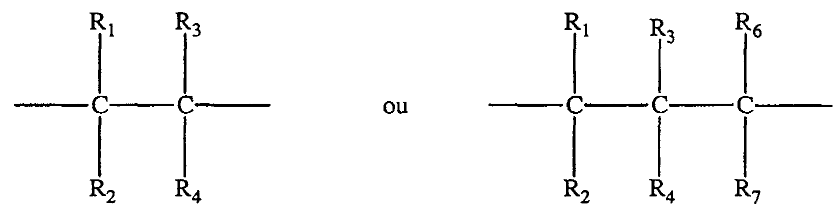 Z représente un atome d'oxygène ou de soufre,
les restes représentés par R₁ à R₄, R₆ et R₇ comportent au plus 20 atomes de carbone et sont chacun, indépendamment l'un de l'autre, un atome d'hydrogène, un groupe alkyle, alcényle, alcynyle, alkaryle, aryle, alicyclique, ou hétéroalicyclique ou hétéroaryle comportant jusqu'à 4 hétéroatomes (les hétéroatomes étant O, N ou S), avec ou sans substituant, deux des groupes R₁ à R₄ et, le cas échéant, R₆ et R₇, séparés par au plus deux atomes de carbone, pouvant éventuellement constituer en commun un système cyclique, lequel système cyclique est un monocycle alicyclique (5 à 8 chaînons) avec ou sans substituant, un monocycle hétéroalicyclique (5 à 8 chaînons dont jusqu'à 2 hétéroatomes [O, N, S]), un bicycle alicyclique (7 à 14 chaînons) ou un bicycle hétéroalicyclique (7 à 14 chaînons dont jusqu'à 4 hétéroatomes [O, N, S]),
et le reste représenté par R₅ comporte au plus 20 atomes de carbone et est un groupe alkyle, alcényle, alcynyle, allyle, alkaryle, aryle ou alicyclique, ou hétéroalicyclique ou hétéroaryle comprenant jusqu'à 4 hétéroatomes (les hétéroatomes étant O, N ou S), avec ou sans substituant.

2. Composés selon la revendication 1, **caractérisés en ce que** les substituants des restes représentés par R₁ à R₇ sont un, deux, trois ou plus des groupes fonctionnels suivants : halogéno, amine tertiaire, nitro, cyano, éther, silyloxyalkyle, thioéther, ester d'acide carboxylique, amide tertiaire d'acide carboxylique, imide d'acide carboxylique, silyle, ester d'acide sulfonique, ester d'acide sulfénique, ester d'acide sulfinique ou sulfonamide tertiaire.

3. Composés selon la revendication 1 ou 2, **caractérisés en ce que** A représente un groupe de formule -C(CH₃)₂-CH₂-.

4. Composés selon l'une quelconque des revendications 1 à 3, à savoir : carbonate d'allyle et de 2-isocyano-2-méthyl-prop-1-yle, carbonate de benzyle et de 2-isocyano-2-méthyl-prop-1-yle, carbonate de 4-nitrobenzyle et de 2-isocyano-2-méthyl-prop-1-yle, carbonate d'éthyle et de 2-isocyano-2-méthyl-prop-1-yle, carbonate de phényle et de 2-isocyano-2-méthyl-prop-1-yle, carbonate de 4-nitrophényle et de 2-isocyano-2-méthylprop-1-yle, carbonate de vinyle et de 2-isocyano-2-méthyl-prop-1-yle, carbonate de 2,2,2-trichloroéthyle et de 2-isocyano-2-méthyl-prop-1-yle, carbonate de 2-triméthylsilyl-éthyle et de 2-isocyano-2-méthyl-prop-1-yle, carbonate de méthyle et de 2-isocyano-2-méthyl-prop-1-yle, carbonate d'allyle et de 1-isocyano-2-méthyl-prop-2-yle, carbonate de benzyle et de 1-isocyano-2-méthyl-prop-2-yle, carbonate de 4-nitrobenzyle et de 1-isocyano-2-méthyl-prop-2-yle, carbonate d'éthyle et de 1-isocyano-2-méthylprop-2-yle, carbonate de phényle et de 1-isocyano-2-méthyl-prop-2-yle, carbonate de 4-nitrophényle et de 1-isocyano-2-méthyl-prop-2-yle, carbonate de vinyle et de 1-isocyano-2-méthyl-prop-2-yle, carbonate de 2,2,2-trichloroéthyle et de 1-isocyano-2-méthyl-prop-2-yle, carbonate de 2-triméthylsilyl-éthyle et de 1-isocyano-2-méthyl-prop-2-yle, carbonate de méthyle et de 1-isocyano-2-méthyl-prop-2-yle.

5. Composés de formules III à IX, 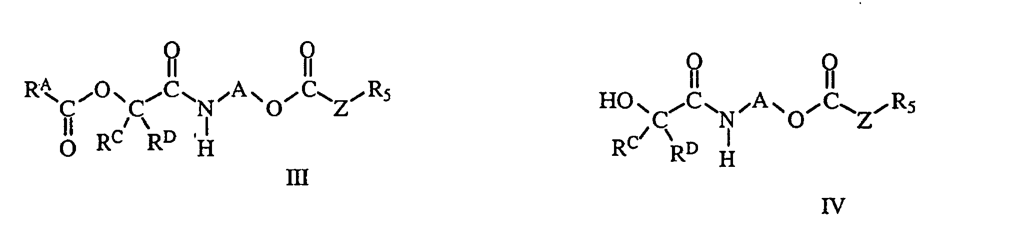 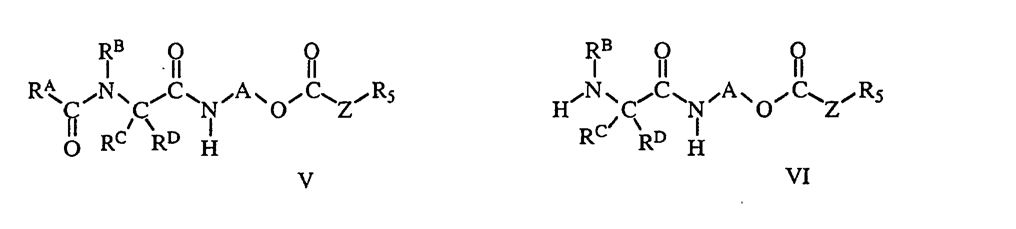 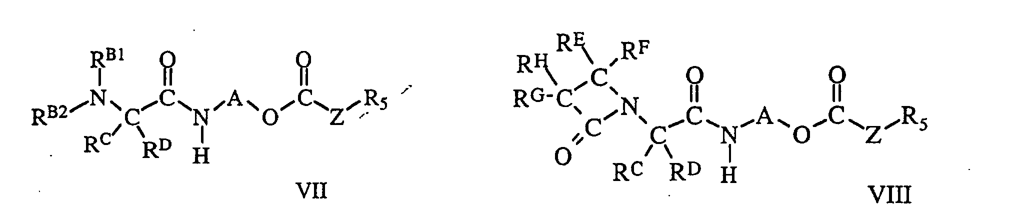 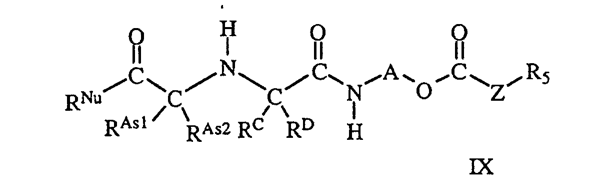 **caractérisés en ce que**
Z, A et R₅ ont les significations indiquées dans la revendication 1 ou 2,
les restes représentés par R^{A}, R^{B}, R^{C}, R^{D}, R^{As1}, R^{As2}, R^{E}, R^{F}, R^{G}, R^{H}, R^{B1} et R^{B2} comprennent chacun jusqu'à 20 atomes de carbone et sont chacun, indépendamment les uns des autres, un atome d'hydrogène, un groupe non substitué de type alkyle, alcényle, alcynyle, alkaryle, aryle, alicyclique ou hétéroalicyclique ou hétéroaryle comprenant jusqu'à 6 hétéroatomes (les hétéroatomes étant O, N ou S), ou un groupe correspondant portant un ou deux substituants fonctionnels ou plus, choisis parmi halogéno, amine tertiaire, nitro, cyano, ester d'acide carboxylique, amide d'acide carboxylique, imide d'acide carboxylique, alcoxy, époxy, boro, silyloxy, silyle, thio, ester d'acide sulfonique, ester d'acide sulfénique, ester d'acide sulfinique, sulfonamide, uréthane ou urée, ou bien R^{B} représente un groupe OH ou RR'N (où R et R' ont, indépendamment l'un de l'autre et de R^{A}, la même signification que R^{A}) ou un reste de 1-aminosucre O-protégé par un groupe alkyle en C₁-C₆ ou (alkyle en C₁-C₆)carbonyle,
et R^{Nu} représente un groupe de formule HO, R^{Alk}O ou R^{Am1}R^{Am2}N, où R^{Alk} représente un groupe méthyle, éthyle, propyle, butyle, allyle ou benzyle, R^{Am1} et R^{Am2} représentent chacun un groupe alkyle en C₁-C₆, ou R^{Am1} et R^{Am2} constituent ensemble un groupe cycloalkyle à 5 ou 6 chaînons ou un groupe cycloalkyle à 6 chaînons comportant un hétéroatome de plus (N ou O).

6. Composés selon l'une quelconque des revendications 1, 2 et 5, **caractérisés en ce qu'**ils sont liés à une phase solide par l'intermédiaire de l'un ou plusieurs des groupes R₁ à R₄, R₆ et R₇, ainsi que R₅ ou Z.

7. Procédé de préparation de composés de formule III ou IV selon la revendication 5 ou 6, **caractérisé en ce qu'**on fait réagir un acide carboxylique R^{A}-COOH ou de l'eau avec un composé carbonylé R^{C}-CO-R^{D} et un derivative d'acide carboxylique d'isocyanoalkyle de formule I, en une réaction de Passerini à trois composants.

8. Procédé de préparation de composés de formule V, VI ou VII selon la revendication 5 ou 6, **caractérisé en ce qu'**on fait réagir un acide carboxylique R^{A}-COOH ou de l'eau avec un composé carbonylé R^{C}-CO-R^{D}, une amine R^{B}-NH₂ ou R^{B1}-NH-R^{B2} et un derivative d'acide carboxylique d'isocyanoalkyle de formule I, en une réaction de Ugi à quatre composants.

9. Procédé de préparation de composés de formule VIII selon la revendication 5 ou 6, **caractérisé en ce qu'**on fait réagir un acide β-aminé H₂N-CR^{E}R^{F}-CR^{G}R^{H}-COOH avec un composé carbonylé R^{C}-CO-R^{D} et un derivative d'acide carboxylique d'isocyanoalkyle de formule I, en une réaction de Ugi à quatre composants avec acide β-aminé.

10. Procédé de préparation de composés de formule IX selon la revendication 5 ou 6, **caractérisé en ce qu'**on fait réagir un acide α-aminé H₂N-CR^{As1}R^{As2}-COOH avec un composé carbonylé R^{C}-CO-R^{D}, un derivative d'acide carboxylique d'isocyanoalkyle de formule I et un nucléophile R^{Nu}-H, en une réaction de Ugi à quatre composants et cinq centres.
